# EUROPÄISCHE PATENTSCHRIFT

(11) **EP 0 865 299 B1**
(45) Veröffentlichungstag und Bekanntmachung des Hinweises auf die Patenterteilung: **26.09.2007**
(21) Anmeldenummer: 96911948.6
(22) Anmeldetag: 27.03.1996
(51) Int. Cl.: A61L 2/06

(54) **GERÄT ZUM REINIGEN UND/ODER DESINFIZIEREN VON CHIRURGISCHEN INSTRUMENTEN**
APPARATUS FOR CLEANSING AND/OR DISINFECTING SURGICAL INSTRUMENTS
APPAREIL DE NETTOYAGE ET/OU DE STERILISATION D'INSTRUMENTS CHIRURGICAUX

(30) Priorität: 28.03.1995 DE 19511037
(43) Veröffentlichungstag der Anmeldung: 23.09.1998
(73) Patentinhaber: Schad, Karl, 78600 Kolbingen (DE)
(72) Erfinder: Schad, Karl, 78600 Kolbingen (DE)
(74) Vertreter: Weiss, Peter
(86) Internationale Anmeldenummer: PCT/EP1996/001333
(87) Internationale Veröffentlichungsnummer: WO 1996/030058

(56) Entgegenhaltungen:
- EP-A- 0 072 257
- WO-A-95/14494
- CA-A- 1 042 622
- DE-A- 4 323 815
- DE-A- 4 443 757
- FR-A- 1 600 886

## Beschreibung

Die Erfindung betrifft ein Verfahren zum Reinigen und/oder Desinfizieren eines chirurgischen Instruments, welches zumindest einen Rohrschaft aufweist, an dem ein Maul angeordnet ist.

Aus der DE 43 23 815 A1 ist ein Verfahren zur hygienischen Aufbereitung von medizinischen, insbesondere zahnmedizinischen Instrumenten mit und ohne Medienkanälen und mit und ohne bewegbaren Innenteilen bekannt, bei dem eine Reinigung der Aussenflächen, eine intensive Nachreinigung und Desinfektion der Aussenflächen und der Medienkanäle sowie der bewegbaren Innenteile und deren Lager, eine Pflege der bewegbaren Innenteilen durch Injektion eines Schmiermittels, eine Sterilisation des Instruments aussen und innen und ein Trocknen und Abkühlen des Instruments stattfindet.

Chirurgische Instrumente sind in vielfältiger Form und Ausführung bekannt und auf dem Markt. Probleme in heutiger Zeit stellen vor allem die gestiegenen Hygieneanforderungen dar. Es ist bekannt, daß durch nichtgereinigte Instrumente Krankheiten weitergegeben werden, was absolut unerwünscht ist, vor allem bei der Gefahr der Weitergabe von Hepatitis und Aids. Deshalb wird heute gefordert, daß chirurgische Instrumente außerordentlich gründlich gereinigt werden. Dies gilt vor allem für solche Instrumente, die aus vielen einzelnen Teilen bestehen, die während des Gebrauchs dieses Instrumentes verschmutzt werden. Meist liegen diese Teile auch an schwer zugänglichen Stellen, so daß ein einfaches Spülen mit Desinfektionsfküssigkeit od.dgl. nicht genügt. Es muß abolut sichergestellt werden, daß entsprechende Bakterien, Viren und sonstige Keime bzw. Erreger abgetötet werden.

Vor allem gilt dies aber für Instrumente der o.g. Art, die zum Einsatz im Körper eines Patienten gelangen. Hierzu zählen beispielsweise Probenexzisionszangen, Instrumente der minimalinvasiven Chirurgie, der Arthroskopie, der Endoskopie usw. Die Aufzählung läßt sich beliebig fortsetzen.

Der vorliegenden Erfindung liegt die Aufgabe zugrunde, ein Verfahren der o.g. Art zu entwickeln, welches diesen gestiegenen Hygieneanforderungen an chirurgische Instrument gerecht wird.

Zur Lösung dieser Aufgabe führen die Merkmale von Anspruch 1.

Das Maul soll mit dem heißen Wasserdampf beaufschlagt und zu einem späteren Zeitpunkt getrocknet werden. Bakterien, Viren und andere Erreger können nur bei einer bestimmten Temperatur existieren. Wird diese Temperatur geändert, so werden auch die Bakterien, Viren und anderen Erreger abgetötet. D.h., es wird im wesentlichen eine Wei- tergabe von Krankheiten vermieden. Die bevorzugte Beaufschlagung mit heißem Dampf und/oder heißer Luft hat den Vorteil, daß beide Medien ein chirurgisches Instrument tief durchdringen können und insbesondere auch an Stellen kriechen, die einer Reinigungsflüssigkeit meist nicht zugänglich sind. Ferner wird durch die Beaufschlagung mit heißem Dampf bzw. Luft das Metall des chirurgischen Instrumentes, sofern dies aus Metall besteht, was zumindest für Maulteile gilt, ebenfalls erhitzt wird, so daß diese Wärme auch an unzugängliche Stellen weitergegeben wird und dort die Krankheitserreger vernichtet werden.

Zur Erzeugung von Dampf bzw. trockener heißer Luft soll den Düsen eine Heizeinrichtung vorgeschaltet sein. In der Regel bietet es sich an, sowohl Wasser als auch Luft in einen Speicher einzufüllen und diesen zu erwärmen. Bei Bedarf wird dann Wasserdampf bzw. heiße Luft dem entsprechenden Speicher entnommen. Möglich ist auch der Anschluß an ein externes Wassernetz.

Die Steuerung der Entnahme von heißem Dampf bzw. heißer Luft geschieht über eine Ventileinrichtung, die bevorzugt mit den übrigen operativen Elementen in einer operativen Kammer angeordnet ist, wobei diese Kammer über eine Mittelwand von der Reinigungskammer getrennt ist. Hierdurch entsteht ein einheitliches Gerät, welches leicht zu transportieren und zu handhaben ist.

Auch der Rohrschaft wird einer besonderen Reinigung und Desinfektion unterworfen, da er auch das Eindringen von Gewebeflüssigkeit, Blut usw. erlaubt. Zu diesen Instrumenten gehören beispielsweise die oben beschriebenen Probenexzisionszangen usw..

Zum Reinigen und Desinfizieren des Rohrschaftinneren und auch der inneren Gelenkteile des Maules usw. soll der Rohrschaft über einen Schlauch an einen Tank für Desinfektionsflüssigkeit angeschlossen werden. Der Rohrschaft kann dann mit dieser Desinfektionsflüssigkeit gespült werden.

Bevorzugt erfolgt die Verbindung zwischen Schlauch und Desinfektionstank ebenfalls über die oben beschriebene Ventileinrichtung, so daß hier je nach Wunsch umgeschaltet werden kann. Es besteht nun auch die Möglichkeit, vor einem Spülen mit Desinfektionsflüssigkeit den Schlauch, der beispielsweise auch Teil des chirurgischen Instrumentes sein kann, mit Wasser bei einem Druck von ca. 8 bar durchzuspülen. Nach dem Desinfizieren mit Desinfektionsflüssigkeit findet ein Ausblasen von Desinfektionsflüssigkeit mit bevorzugt heißer Luft statt.

Da bei dem erfindungsgemäßen Verfahren Medien, sei es Wasser, Wasserdampf oder Luft bzw. Desinfektionsflüssigkeit unter Druck gesetzt werden, ist in dem operativen Teil des Gerätes noch eine oder mehrere Pumpen integriert. Sie sind gleichfalls mit der Ventileinrichtung verbunden, so daß sie je nach Bedarf zugeschaltet werden.

Eine Verbesserung der Reinigung bzw. Desinfektion des chirurgischen Instrumentes wird noch dadurch erreicht, daß das Instrument gegenüber den Düsen bzw. die Düsen gegenüber dem Instrument gedreht wird/werden. Hierdurch kann der Wasserdampf bzw. die trockene Luft an unterschiedlichste Stellen des chirurgischen Instrumentes gelangen. Ein Ausführungsbeispiel einer einfachen Dreheinrichtung besteht aus einer Drehscheibe, in welcher das chirurgische Instrument sitzt. Gegebenenfalls kann hier das chirugische Instrument noch von einer Halterung umgeben sein, die eine Anpassung der Drehscheibe an unterschiedlich ausgestaltete chirurgische Instrumente zuläßt.

Die Drehscheibe ist dann mit einem Antrieb verbunden. Eine Drehbewegung wird beispielsweise über ein Zahnrad von dem Antrieb auf eine Außenzahnung der Drehscheibe übertragen. Hier sind viele Ausführungsformen denkbar und sollen von der Erfindung umfaßt sein.

Durch das vorliegende erfindungsgemäße Verfahren wird die Reinigung und Desinfektion von chirurgischen Instrumenten automatisiert, erleichtert und wesentlich verbessert. Das Gerät arbeitet nach Programmen ähnlich einer Spülmaschine, je nach dem chirurgischen Instrument bzw. den Anforderungen an die Reinigung und Desinfektion.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Frontansicht eines Gerätes zum Reinigen und Desinfizieren von chirurgischen Instrumenten;
Figur 2 einen Querschnitt durch ein Element des Gerätes nach Figur 1 entlang Linie II - II.

Ein Gerät R zum Reinigen und Desinfizieren von chirurgischen Instrumenten 1, insbesondere von Probenexzisionszangen, weist ein Gehäuse 2 auf. Dieses Gehäuse 2 ist durch eine Mittelwand 3 in eine Reinigungskammer 4 und eine operative Kammer 5 unterteilt. Zu der Reinigungskammer 4 gehört auch eine Auffangwanne 6 zum Auffangen von kondensiertem Dampf bzw. Desinfizierflüssigkeit.

Das chirurgische Instrument 1 weist ein Maul 7 an einem Rohrschaft 8 auf. Dieser Rohrschaft 8 ist in eine Halterung 9 eingebettet und besitzt andererseits einen flexiblen Schlauch 10, der mit einem Anschluß 11 an die operative Kammer 5 angekoppelt ist. Der Schlauch 10 kann aus einem beliebigen flexiblen Material, beispielsweise Kunststoff, hergestellt sein. Es gibt Instrumente, bei denen der Schlauch 10 Bestandteil des chirurgischen Instrumentes 1 ist, um beispielsweise Gewebeproben aus dem Körper zu einem Aufnahmebehälter abzuleiten. Es gibt auch chirurgische Instrumente, die keinen derartigen Schlauch 10. besitzen. Hier ist dann der Schlauch 10 lediglich als Verbindungsleitung zwischen dem chirurgischen Instrument 1 und dem Anschluß 11 ausgebildet.

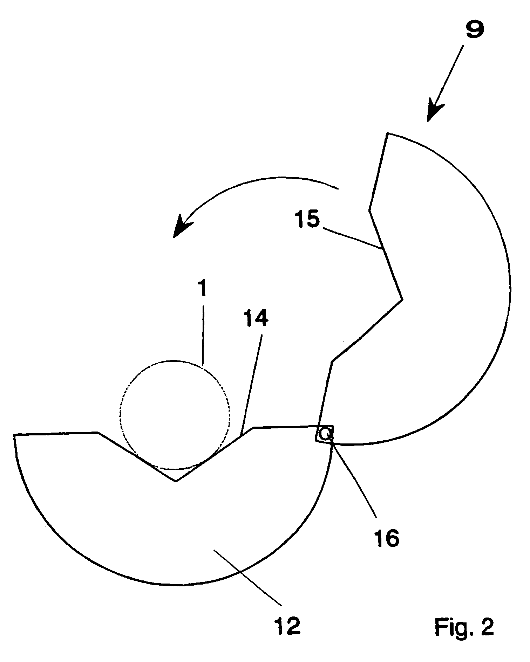

Die Halterung 9 besteht gemäß Figur 2 der Einfachheit halber aus zwei Halbschalen 12 und 13, die jeweils eine Längsnut 14 und 15 zur Aufnahme des chirurgischen Instrumentes 1 besitzen. Beide Halbschalen 12 und 13 sind über ein Gelenk 16 miteinander verbunden. Die Längsnuten 14 und 15 weisen bevorzugt eine keilförmige Form auf, so daß sie in der Lage sind, chirugische Instrumente 1 mit unterschiedlichen Rohrschaftdurchmessern aufzunehmen. Ferner ist es auch denkbar, die Längsnuten 14 und 15 mit elastischem Material auszukleiden, um eine bessere Halterung von chirurgischen Instrumenten 1 mit unterschiedlichen Rohrschaftdurchmessern zu erzielen.

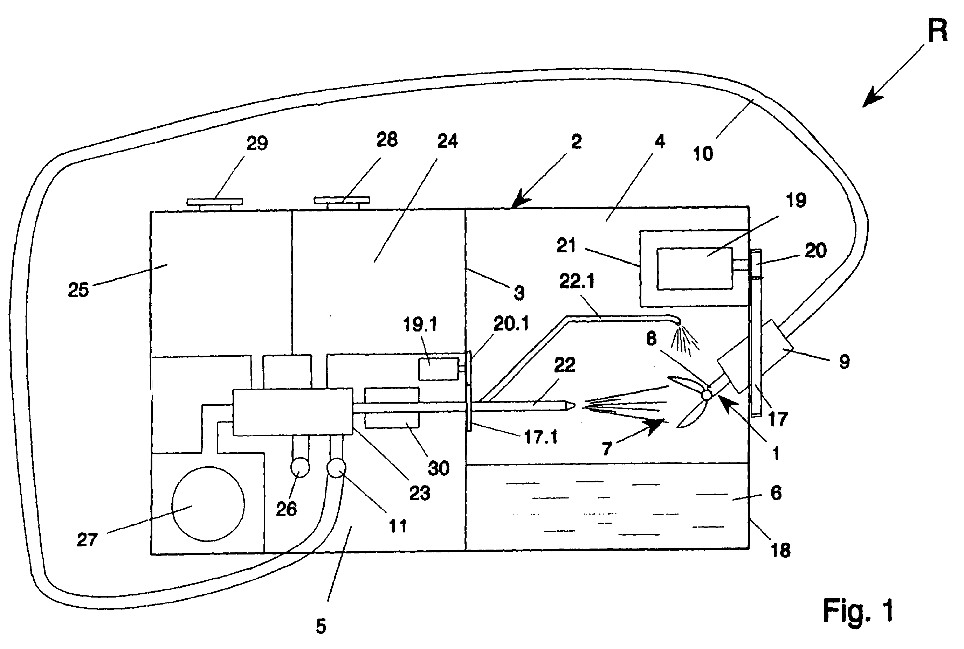

In Gebrauchlage (s. Figur 1) steckt die Halterung 9 in einer Drehscheibe 17, die drehbar an einer Außenwand 18 des Gehäuses 2 angeordnet ist. Dabei ist die Drehscheibe 17 mit einem Antrieb 19 verbunden, der die Drehscheibe 17 drehen kann. Der Einfachheit halber wird eine Verbindung zwischen dem Antrieb 19 und der Drehscheibe 17 über ein Zahnrad 20 bewirkt, welches in eine Außenzahnung in der Drehscheibe 17 eingreift. Im vorliegenden Ausführungsbeispiel befindet sich der Antrieb 19 in der Reinigungskammer 4 und ist deshalb durch eine Abdeckung 21 gekapselt.

In der Reinigungskammer 4 sind dem chirurgischen Instrument 1 und insbesondere dem Maul 7 zwei Düse 22.1 und 22.2 zugeordnet, über welche das Maul 7 mit Dampf, Desinfizierflüssigkeit und/oder Luft beaufschlagt werden kann. Diese Düsen sind mittels ähnlicher Einrichtungen (17.1, 19.1, 20.1) drehbar ausgestaltet, wie die Halterung 9.

Innerhalb der operativen Kammer 5 sind die Düsen 22 mit einer nur schematisch angedeuteten Ventileinrichtung 23 verbunden. Mit dieser Ventileinrichtung 23 sind ferner ein Wassertank 24, ein Tank 25 für eine Desinfektionsflüssigkeit, ein Druckluftanschluß 26 nach außen oder an einen integrierten Lufterzeuger und eine Pumpe 27 gekoppelt. Die Ventileinrichtung 23 kann sowohl die Düsen 22 als auch den Anschluß 11 über den Schlauch 10 zu dem chirurgischen Instrument 1 mit dem Wassertank 24, dem Desinfektionstank 25 oder dem Druckluftanschluß 26 verbinden. Innerhalb der Ventileinrichtung 23 wird der notwendige Druck über die Pumpe 27 aufrechterhalten.

Wassertank 24 und Desinfektionstank 25 sind über entsprechende Deckel 28 und 29 verschlossen.

Zum Reinigen und Desinfizieren wird das chirurgische Instrument 1 in die Halterung 9 eingelegt. Dabei liegt der Rohrschaft 8 in den entsprechenden Längsnuten 14 und 15. Sofern das chirurgische Instrument 1 keinen flexiblen Schlauch 10 aufweist, wird es rückwärtig an den Schlauch 10 des Gerätes R angeschlossen. Ansonsten erfolgt eine Festlegung des Schlauches 10 an dem Anschluß 11.

Nunmehr wird die Halterung 9 in die Drehscheibe 17 eingeschoben, in welcher die Halterung 9 form- und/oder kraftschlüssig gehalten ist. Beispielsweise kann sich in der Drehscheibe 17 eine Ausnehmung befinden, die mit einem gummiartigen Material ausgekleidet ist. Dieses gummiartige Material umschließt dann die Halterung 9 eng, so daß die Halterung 9 gegenüber der Drehscheibe 17 nur unter erhöhtem Kraftauwand gedreht werden kann.

In dieser Gebrauchslage befindet sich das Maul 7 des chirurgischen Instrumentes 1 nahe den Düsen 22. Nunmehr erfolgt eine Beaufschlagung des Maules 7 über die Düsen 22 mit heißem Wasserdampf von ungefähr 120°C. Während dieser Dampfbeaufschlagung sollte das chirurgische Instrument 1 gedreht werden. Hierzu wird der Antrieb 19 betätigt, der die Drehscheibe 17 bevorzugt zweimal rechts und dann zweimal links dreht. Dadurch wird das Maul von allen Seiten mit Dampf beaufschlagt. Hierdurch werden das Maul und insbesondere die Gelenkteile wirkungsvoll gereinigt und desinfiziert.

Danach verbindet die Ventileinrichtung 23 den Desinfektionstank 25 mit dem Anschluß 11, so daß das gesamte chirurgische Instrument 1 mit Desinfektionsflüssigkeit über den Schlauch 10 gespült und gereinigt werden kann.

Nach dem Spülen mit Desinfektionsflüssigkeit soll etwas abgewartet werden, um die Desinfektionsflüssigkeit in allen Bereichen des chirurgischen Instrumentes 1 wirken zu lassen. Nunmehr verbindet die Ventileinrichtung 23 den Anschluß 11 mit dem Druckluftanschluß 26. Durch den Schlauch 10 wird das chirurgische Instrument 1 mit Druckluft ausgeblasen, so daß sich in dem chirurgischen Instrument 1 keine Desinfektionsflüssigkeit mehr befindet. Möglich ist auch ein vorgängiges Spülen mit Wasser.

Danach erfolgt ein Verbinden des Druckluftanschlusses 26 mit den Düsen 22, wobei eine Heizeinrichtung 30 die Luft vor den Düsen 22 erwärmt. D.h., das Maul 7 wird nun durch Heißluft getrocknet. Auch dabei kann das Instrument 1 wieder gedreht werden.

Es versteht sich von selbst, daß der gesamte Reinigungs- und Desinfektionsvorgang möglichst vollautomatisch ablaufen soll, d.h. dem Gerät R ist eine entsprechende elektronische Schaltung mit Regelautomatik zugeordnet. Diese ist der Einfachheit halber in den Zeichnungen vernachlässigt.

## Patentansprüche

1. Verfahren zum Reinigen und/oder Desinfizieren eines chirurgischen Instrumentes (1), welches zumindest einen Rohrschaft (8) aufweist, an dem ein Maul (7) angeordnet ist, wobei das Maul (7) mit heissem Dampf beaufschlagt und der Rohrschaft mit Wasser und/oder Desinfektionsflüssigkeit und/oder Dampf gespült wird.

2. Verfahren nach Anspruch 1, **dadurch gekennzeichnet, dass** das Maul (7) des chirurgischen Instrumentes (1) geöffnet ist.

3. Verfahren nach Anspruch 1 oder 2, **dadurch gekennzeichnet, dass** der Rohrschaft (8) nach dem Spülen mit heisser Luft getrocknet und das Maul mit trockener heisser Luft beaufschlagt wird.

4. Verfahren nach einem der Ansprüche 1-3, **dadurch gekennzeichnet, dass** das chirurgische Instrument (1) und/oder eine Düse (22.1, 22.2) zum Aufbringen von Dampf während dem Reinigen und/oder Desinfizieren gedreht wird.

## Claims

1. Method of cleaning and/or disinfecting a surgical instrument (1), which comprises at least one tubular shank (8), on which a mouth (7) is disposed, whereby the mouth (7) is acted upon by hot steam and the tubular shank is rinsed with water and/or liquid disinfectant and/or steam.

2. Method according to claim 1, **characterized in that** the mouth (7) of the surgical instrument (1) is open.

3. Method according to claim 1 or 2, **characterized in that** the tubular shank (8) after rinsing is dried with hot air and the mouth is acted upon by dry hot air.

4. Method according to one of claims 1 - 3, **characterized in that** the surgical instrument (1) and/or a nozzle (22.1, 22.2) for applying steam is rotated in the course of cleaning and/or disinfecting.

## Revendications

1. Procédé pour nettoyer et/ou désinfecter un instrument chirurgical (1), qui présente au moins un manche tubulaire (8) sur lequel est disposé un bec (7), le bec (7) étant soumis à de la vapeur chaude et le manche tubulaire étant rincé avec de l'eau et/ou du liquide désinfectant et/ou de la vapeur.

2. Procédé selon la revendication 1, **caractérisé par le fait que** le bec (7) de l'instrument chirurgical (1) est ouvert.

3. Procédé selon la revendication 1 ou 2, **caractérisé par le fait que** le manche tubulaire (8) est, après le rinçage, séché par de l'air chaud et le bec est soumis à de l'air chaud sec.

4. Procédé selon l'une des revendications 1 à 3, **caractérisé par le fait que** l'instrument chirurgical (1) et/ou une tuyère (22.1, 22.2) est tourné pour l'application de vapeur pendant le nettoyage et/ou la désinfection.
